# EUROPÄISCHE PATENTANMELDUNG

(11) **EP 2 272 457 A1**
(43) Veröffentlichungstag der Anmeldung: **12.01.2011**
(21) Anmeldenummer: 10006943.4
(22) Anmeldetag: 06.07.2010
(51) Int. Cl.: A61B 19/02, A61B 19/00

(54) **Haltevorrichtung für einen Abfallbehälter**

(30) Priorität: 07.07.2009 DE 102009032170
(71) Anmelder: Soehnle Professional GmbH & Co. KG, 71522 Backnang (DE)
(72) Erfinder: Gerster, Stephan, 53343 Wachtberg-Pech (DE)
(74) Vertreter: Hoffmann, Jürgen

(57) **Zusammenfassung**

Bei einer Haltevorrichtung an und/oder auf der ein Abfallbehälter, insbesondere ein Einwegbehälter zur Entsorgung von klinischem Abfall, wieder lösbar befestigbar ist, ist eine Erkennungsvorrichtung vorgesehen, die Art und/oder Typ eines an der Haltevorrichtung befestigten Abfallbehälters erkennt; dies insbesondere dadurch, dass der Abfallbehälter eine mechanische Kodierung seiner Art und/oder seines Typs aufweist.

## Beschreibung

Die Erfindung betrifft eine Haltevorrichtung an und/oder auf der ein Abfallbehälter, insbesondere ein Einwegbehälter zur Entsorgung von klinischem Abfall, wieder lösbar befestigbar ist. Die Erfindung betrifft außerdem einen Abfallbehälter zur Befestigung an einer solchen Haltevorrichtung.

Abfallbehälter, insbesondere Einwegabfallbehälter und solche zur Entsorgung von Klinikabfällen, sind seit geraumer Zeit bekannt und in unterschiedlichsten Arten und Typen auf dem Markt erhältlich. Um ein versehentliches Umstoßen oder Umfallen solcher Abfallbehälter zu verhindern, wird in DE 91 12 444.9 vorgeschlagen einen Halter zu verwenden, der beispielsweise als Saughalter ausgebildet sein kann und eine wieder lösbare Verbindung zu einem Tisch oder zum Boden herstellt.

Es ist darüber hinaus bekannt, dass nicht jede Art oder nicht jeder Typ von Abfallsammler für jegliche Abfälle verwendet werden kann. Im Gegenteil sind insbesondere bei kritischen Abfällen, wie beispielsweise bei unsterilen und/oder spitzen und/oder scharfkantigen Klinikabfällen besondere Anforderungen an den Abfallbehälter zu stellen. Bei Verwendung eines ungeeigneten Abfallbehälters kann es zu schwerwiegenden Verletzungen, beispielsweise durch durch die Außenhülle dringende Kanülen oder Skalpelle kommen. Es besteht auch die Gefahr, dass das Material des Abfallbehälters durch den Abfall chemisch angegriffen wird.

Es ist daher die Aufgabe der vorliegenden Erfindung, eine Haltevorrichtung und einen entsprechenden Abfallbehälter anzugeben, mit dem der versehentliche Einsatz von Abfallbehältern ungeeigneter Art oder ungeeigneten Typs besser vermieden werden können.

Die Aufgabe wird durch eine Haltevorrichtung gelöst, die dadurch gekennzeichnet ist, dass eine Erkennungsvorrichtung vorgesehen ist, die Art und/oder Typ eines an der Haltevorrichtung befestigten Abfallbehälters erkennt.

Die erfindungsgemäße Haltevorrichtung hat den Vorteil, dass sie Art und Typ des befestigten Abfallbehälters erkennt und demgemäß dem Benutzer Hinweise oder Warnungen ausgeben kann.

Insbesondere kann in erfindungsgemäßer Weise eine Ausgabevorrichtung vorgesehen sein, die Art und/oder Typ des befestigten Behälters optisch und/oder akustisch wahrnehmbar anzeigt. Beispielsweise kann die Ausgabevorrichtung Art oder Typ des befestigten Abfallbehälters per Sprachausgabe dem Benutzer mitteilen. Alternativ oder zusätzlich kann vorgesehen sein, dass die Ausgabevorrichtung Art oder Typ des befestigten Behälters auf einem Display graphisch oder in Form von Schriftzeichen oder in Form eines Nummernkodes angibt. Insbesondere kann vorgesehen sein, dass die Ausgabevorrichtung den Benutzer warnt, wenn ein Abfallbehälter ungeeigneten Typs an der Haltevorrichtung befestigt ist.

Bei einer besonderen Ausführungsform ist vorgesehen, dass die Haltevorrichtung den zunächst befestigten Abfallbehälter automatisch löst, wenn erkannt worden ist, dass der Abfallbehälter nach Art oder Typ ungeeignet für die vorgesehene Anwendung sein könnte.

Bei einer besonderen Ausführungsform der erfindungsgemäßen Haltevorrichtung weist diese eine Wiegevorrichtung und/oder eine Kraftmessvorrichtung auf. Mit Hilfe der Wiegevorrichtung und/oder der Kraftmessvorrichtung kann beispielsweise eine Überladung des Abfallbehälters detektiert werden. Es ist alternativ oder zusätzlich auch möglich, eine von außen auf einen befestigten Haltebehälter wirkende Krafteinwirkung zu detektieren. Diese Ausführungsform hat den Vorteil, dass eine Warnung ausgegeben werden kann, wenn der möglicherweise scharfkantige oder spitze Abfall vom Benutzer zusammengedrückt und komprimiert wird, um im eigentlich schon gefüllten Abfallbehälter zusätzlichen Platz für weiteren Abfall zu schaffen. Diese Warnfunktion vermeidet schwerwiegende Verletzungen an der pressenden Hand oder dem pressenden Fuß. Darüber hinaus wird wirkungsvoll vermieden, dass durch ein eigentlich unzulässiges Zusammenpressen spitze oder scharfkantige Gegenstände gewaltsam durch die Abfallbehälterwand nach außen gestoßen werden.

Bei einer ganz besonders vorteilhaften Ausführungsform ist vorgesehen, dass die Erkennungsvorrichtung Art und/oder Typ des Abfallbehälters an zumindest einem Teil der Form des Abfallbehälters erkennt. Beispielsweise kann die Erkennungsvorrichtung zumindest einen Fühler und/oder zumindest einen Tastschalter aufweisen, der zumindest einen Teil der Form des befestigten Abfallbehälters abtastet. Insbesondere kann der Tastschalter und/oder der Fühler eine vordefinierte Form aufweisen, die zumindest einen mechanischen Kode des Abfallbehälters korrespondiert.

Bei einer besonders vorteilhaften Ausführungsform liest die Erkennungsvorrichtung eine mechanische Kodierung von einem befestigten Abfallbehälter - vorzugsweise durch Abtasten - ab.

Beispielsweise kann die Erkennungsvorrichtung einer Reihe von Tastköpfen oder eine Matrix von Tastköpfen aufweisen, die von einem auf dem Abfallbehälter angeordneten, seinen Typ und/oder seine Art repräsentierenden Muster, unterschiedlich verschoben oder beaufschlagt werden.

Es kann auch vorgesehen sein, dass die Erkennungsvorrichtung eine Tastvorrichtung zum Erkennen einer Wölbung und/oder eines Wölbungsradius aufweist. Demgemäß kann ein entsprechender Abfallbehälter derart ausgebildet sein, dass seine Art und/oder sein Typ in der Tiefe einer Wölbung und/oder in einem Wölbungsradius kodiert ist. Beispielsweise könnte der Abfallbehälter einen - vorzugsweise nach innen - gewölbten Boden aufweisen.

Bei einer anderen besonderen Ausführungsform weist die Erkennungsvorrichtung eine Tastvorrichtung zum Erkennen einer Öffnung und/oder Ausnehmen und/oder eines Öffnungsdurchmessers und/oder eines Ausnehmungsdurchmessers auf. Demgemäß könnte ein entsprechender Abfallbehälter eine Öffnung oder Ausnehmung aufweisen, in deren Größe und Tiefe die Art und/oder der Typ des Abfallbehälters kodiert sind.

Bei einer weiteren erfindungsgemäßen Haltevorrichtung weist die Erkennungsvorrichtung eine Tastvorrichtung zum Erkennen der Position einer oder mehrerer Öffnungen oder Ausnehmungen oder der Position beziehungsweise der Positionen von hervorstehenden Teilen auf. Entsprechend könnte der Abfallbehälter derart ausgebildet sein, dass Art und/oder Typ des Abfallbehälters in der Position einer Öffnung oder Ausnehmung oder eines hervorstehenden Teiles beziehungsweise in den Positionen mehrerer Öffnungen, Ausnehmungen oder hervorstehender Teile kodiert sind.

Grundsätzlich sind verschiedenste Arten der mechanischen Kodierung, insbesondere in der äußeren Form des Abfallbehälters, denkbar. Besonders vorteilhaft sind solche Kodierungen, die die übrigen Eigenschaften und Vorteile des Abfallbehälters nicht einschränken. Demgemäß kann die Kodierung, insbesondere die mechanische Kodierung, im und/oder am Boden des Abfallbehälters vorgesehen sein. An dieser Stelle ist die Kodierung gut vor Beschädigungen geschützt. Es kann alternativ oder zusätzlich auch vorgesehen sein, dass Art und/oder Typ des Abfallbehälters innerhalb eines Schraub- oder Bajonettanschlusses - vorzugsweise mechanisch abtastbar - kodiert sind.

Bei einer besonderen Ausführungsform ist eine längsverschiebbare Kugel als Taster vorgesehen, die auf der gedachten Mittelachse eines Schraub- oder Bajonettanschlusses angeordnet ist. Entsprechend sind die befestigbaren Abfallbehälter, je nach Art und Typ, in der Mitte des Bodens mit unterschiedlichen Ausnehmungen versehen, in die der Kugeltaster je nach Öffnungsdurchmesser der Ausnehmung und je nach Tiefe der Ausnehmung beim Befestigen des Abfallbehälters auf der Haltevorrichtung unterschiedlich tief eindringt. Je nach Eindringtiefe erkennt die Erkennungsvorrichtung um welche Art und/oder um welchen Typ von Abfallbehälter es sich handelt.

Bei einer anderen Ausführungsform ist eine Vielzahl von längsverschiebbaren matrixartig angeordneten Tastern vorgesehen, die ein Muster von Ausnehmungen am Boden eines Abfallbehälters eines Abfallbehälters abtasten. Hierbei kann in einfacher Weise durch die Erkennungsvorrichtung ermittelt werden, ob ein Taster durch den Boden des Abfallbehälters gedrückt ist oder nicht gedrückt ist, weil er vollständig in eine Ausnehmung eingreift. Insoweit ist Art und Typ des befestigten Abfallbehälters auf mechanischer Weise binär kodiert und durch die Matrix von Tastköpfen ablesbar.

In der Zeichnung ist der Erfindungsgegenstand schematisch dargestellt und wird anhand der Figuren nachfolgend beschrieben, wobei gleich wirkende Elemente mit denselben Bezugszeichen versehen sind. Dabei zeigen:
- Fig.1: eine erfindungsgemäße Haltervorrichtung mit einem erfindungsgemäßen Abfallbehälter und
- Fig.2: eine andere erfindungsgemäße Haltervorrichtung mit einem entsprechenden Abfallbehälter.

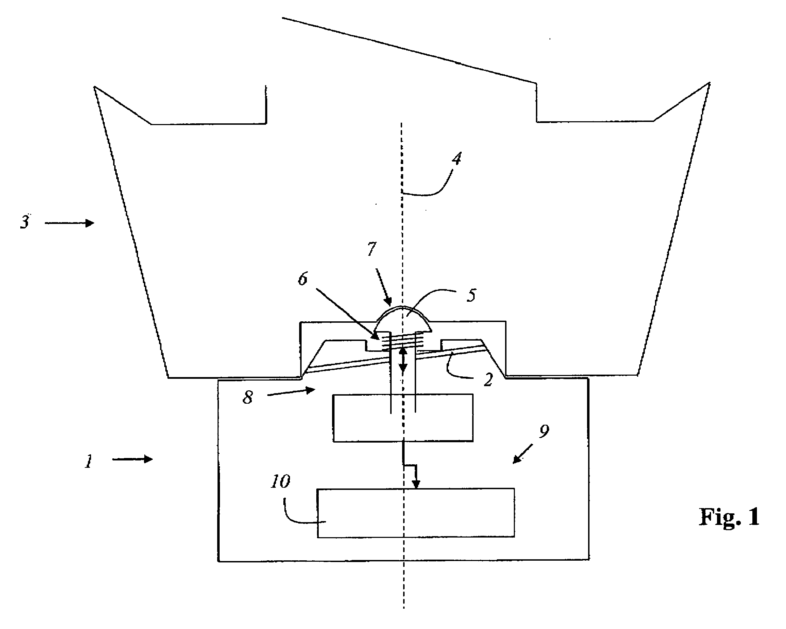

Fig. 1 zeigt eine erfindungsgemäße Haltevorrichtung 1, die mit Hilfe eines nicht gezeigten Saugnapfs, beispielsweise auf einem Tisch, befestigt werden kann. An der Oberseite weist die Haltevorrichtung ein Außengewinde 2 auf, auf das ein Abfallbehälter 3 durch Drehen um eine Schraubachse 4 aufgeschraubt werden kann. Auf der gedachten Schraubachse 4 befindet sich kalottenförmiger Tastkopf 5, der mit Hilfe einer Feder 6 in Richtung auf den aufgesetzten Abfallbehälter 3 gedrückt wird. Der Abfallbehälter 3 weist am Boden eine kugelförmig gewölbte Ausnehmung 7 auf, wobei Art und Typ des Abfallbehälters durch die Tiefe der Ausnehmung kodiert sind. Der Tastkopf 5 ist Bestandteil einer Tastvorrichtung 8, die die Eindringtiefe des Tastkopfes 5 in die Ausnehmung 7 ermittelt und anhand einer elektronisch abgelegten Tabelle Art und Typ des befestigten Abfallbehälter ermittelt und entsprechende Daten zur Anzeige von Art und Typ an eine Anzeigevorrichtung 9, die als Display 10 ausgebildet ist, leitet. Auf dem Display 10 kann der Benutzer ablesen, für welche Art von Abfall der Abfallbehälter geeignet ist und welches Füllvolumen der Abfallbehälter besitzt.

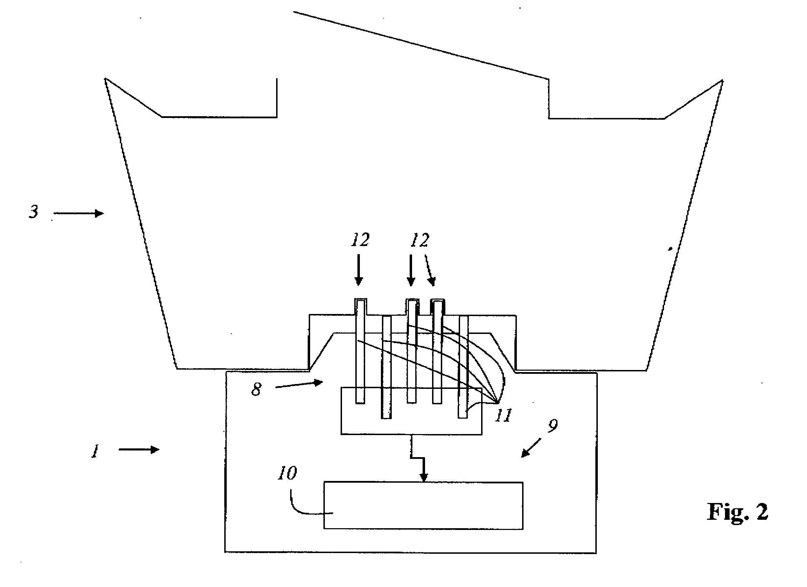

Fig. 2 zeigt eine andere erfindungsgemäße Haltevorrichtung, die mehrere Tastköpfe 11 aufweist, um einen am Abfallbehälter angebrachten mechanischen Kode abzulesen. Der am Abfallbehälter angebrachte Kode ist ein Binärkode, bei dem eine 0 in Form einer Ausnehmung 12 repräsentiert ist, während eine 1 durch eine glatte Oberfläche - dem gemäß also durch eine fehlende Ausnehmung - repräsentiert ist. Die Erkennungsvorrichtung ermittelt demgemäß, welche Tastköpfe 11 in Ausnehmungen 12 eingedrungen sind und welche Tastköpfe nicht. Durch Vergleich des abgetasteten Musters mit elektronisch gespeicherten, Daten ermittelt die Erkennungsvorrichtung Art und Typ des befestigten Abfallbehälters und leitet diese Information optisch oder akustisch wahrnehmbar dem Benutzer zu.

Die Erfindung wurde in Bezug auf eine besondere Ausführungsform beschrieben. Es ist jedoch selbstverständlich, dass Änderungen und Abwandlungen durchgeführt werden können, ohne dabei den Schutzbereich der nachstehenden Ansprüche zu verlassen.

### Bezugszeichenliste

- 1: Haltevorrichtung
- 2: Gewinde
- 3: Abfallbehälter
- 4: Schraubachse
- 5: Tastkopf
- 6: Feder
- 7: Ausnehmung
- 8: Tastvorrichtung
- 9: Anzeigevorrichtung
- 10: Display
- 11: Tastköpfe
- 12: Ausnehmungen

## Patentansprüche

1. Haltevorrichtung an und/oder auf der ein Abfallbehälter, insbesondere ein Einwegbehälter zur Entsorgung von klinischem Abfall, wieder lösbar befestigbar ist, **dadurch gekennzeichnet, dass** eine Erkennungsvorrichtung vorgesehen ist, die Art und/oder Typ eines an der Haltevorrichtung befestigten Abfallbehälters erkennt.

2. Haltevorrichtung nach Anspruch 1, **dadurch gekennzeichnet, dass** eine Ausgabevorrichtung vorgesehen ist, die Art und/oder Typ des befestigten Behälters optisch und/oder akustisch wahrnehmbar anzeigt und/oder dass eine Ausgabevorrichtung vorgesehen ist, die optisch und/oder akustisch warnt, wenn ein Abfallbehälters ungeeigneten Typs an der Haltevorrichtung befestigt ist.

3. Haltevorrichtung nach einem der Ansprüche 1 oder 2, **dadurch gekennzeichnet, dass** die Haltevorrichtung eine Wiegevorrichtung und/oder Kraftmessvorrichtung aufweist.

4. Haltevorrichtung nach Anspruch 3, **dadurch gekennzeichnet, dass** die Haltevorrichtung mit der Wiegevorrichtung und/oder die Kraftmessvorrichtung eine Überladung des Abfallbehälter detektiert und/oder dass die Haltevorrichtung mit der Wiegevorrichtung und/oder die Kraftmessvorrichtung eine von außen auf einen befestigten Abfallbehälter wirkende Krafteinwirkung detektiert.

5. Haltevorrichtung nach Anspruch 4, **dadurch gekennzeichnet, dass** eine Ausgabevorrichtung vorgesehen ist, die eine Überladung und/oder eine Krafteinwirkung von außen auf einen befestigten Abfallbehälter optisch und/oder akustisch wahrnehmbar anzeigt.

6. Haltevorrichtung nach Anspruch einem der Ansprüche 1 bis 5, **dadurch gekennzeichnet, dass** die Erkennungsvorrichtung Art und/oder Typ an zumindest einem Teil der Form des Abfallbehälters erkennt.

7. Haltevorrichtung nach einem der Ansprüche 1 bis 6, **dadurch gekennzeichnet, dass** die Erkennungsvorrichtung zumindest einen Fühler und/oder zumindest einen Tastschalter aufweist, der zumindest einen Teil der Form des befestigten Abfallbehälters abtastet.

8. Haltevorrichtung nach Anspruch 7, **dadurch gekennzeichnet, dass** der Tastschalter und/oder der Fühler eine vordefinierte Form aufweist, die zu zumindest einem mechanischen Kode des Abfallbehälters korrespondiert.

9. Haltevorrichtung nach einem der Ansprüche 1 bis 8, **dadurch gekennzeichnet, dass** die Erkennungsvorrichtung eine mechanische Kodierung von einem befestigten Abfallbehälter - vorzugsweise durch Abtasten - abliest.

10. Haltevorrichtung nach einem der Ansprüche 1 bis 9, **dadurch gekennzeichnet, dass** die Erkennungsvorrichtung eine Tastvorrichtung zum Erkennen einer Wölbung und/oder eines Wölbungsradius aufweist und/oder dass die Erkennungsvorrichtung eine Tastvorrichtung zum Erkennen einer Öffnung und/oder Ausnehmung und/oder eines Öffnungsdurchmessers und/oder Ausnehmungsdurchmessers aufweist und/oder dass die Erkennungsvorrichtung eine Tastvorrichtung zum Erkennen der Position einer Öffnung oder Ausnehmung oder eines hervorstehenden Teiles aufweist und/oder dass die Erkennungsvorrichtung eine Tastvorrichtung zum Erkennen eines mechanischen Kodemusters aufweist.

11. Abfallbehälter, insbesondere Einwegbehälter zur Entsorgung von klinischem Abfall, zur Befestigung an einer Haltevorrichtung nach einem der Ansprüche 1 bis 10.

12. Abfallbehälter nach Anspruch 11, **dadurch gekennzeichnet, dass** Art und/oder Typ des Abfallbehälters in der Form - insbesondere in der äußeren Form - des Abfallbehälters kodiert sind und/oder dass Art und/oder Typ des Abfallbehälters in der Form des Abfallbehälters mechanisch abtastbar kodiert sind und/oder dass Art und/oder Typ des Abfallbehälters im und/oder am Boden des Abfallbehälters - vorzugweise mechanisch abtastbar - kodiert sind und/oder dass Art und/oder Typ des Abfallbehälters innerhalb eines Bajonettanschlusses - vorzugweise mechanisch abtastbar - kodiert sind.

13. Abfallbehälter nach einem der Ansprüche 11 oder 12, **dadurch gekennzeichnet, dass** der Abfallbehälter einen gewölbten Boden aufweist oder dass der Abfallbehälter einen nach innen gewölbten Boden aufweist.

14. Abfallbehälter nach Anspruch 13, **dadurch gekennzeichnet, dass** Art und/oder Typ des Abfallbehälters im Wölbungsradius und/oder in der Tiefe der Wölbung und/oder im Öffnungsdurchmesser der Wölbung kodiert sind und/oder dass der Abfallbehälter eine Ausnehmung aufweist, in deren Größe und/oder Tiefe die Art und/oder der Typ des Abfallbehälters kodiert ist und/oder dass Art und/oder der Typ des Abfallbehälters in der Position einer Öffnung oder Ausnehmung oder eines hervorstehenden Teiles kodiert sind.

15. Abfallentsorgungsvorrichtung mit einer Haltevorrichtung nach einem der Ansprüche 1 bis 10 und einem Abfallbehälter nach einem der Ansprüche 11 bis 14.
